# DEMANDE DE BREVET EUROPEEN

(11) **EP 4 026 578 A1**
(43) Date de publication de la demande: **13.07.2022**
(21) Numéro de dépôt: 21215556.8
(22) Date de dépôt: 17.12.2021
(51) Int. Cl.: A61M 16/00, A61B 5/083, A61B 5/087, A61B 5/00

(54) **BOITIER AUTONOME DE MONITORAGE D'UNE VENTILATION DÉLIVRÉE PENDANT UNE RÉANIMATION CARDIO-PULMONAIRE**

(30) Priorité: 08.01.2021 FR 2100155
(71) Demandeur: Air Liquide Medical Systems, 92182 Antony Cedex (FR)
(72) Inventeur: RICHARD, Jean-Christophe, 92182 Antony Cedex (FR); BADAT, Bilal, 92182 Antony Cedex (FR); PROUVEZ, Nathan, 92182 Antony Cedex (FR); HANNOUCENE, Manon, 92182 Antony Cedex (FR); LESIMPLE, Arnaud, 92182 Antony Cedex (FR); DERMEL, Marius, 92182 Antony Cedex (FR)
(74) Mandataire: Air Liquide

(57) **Abrégé**

L'invention concerne un dispositif de monitorage et de diagnostic (1) d'une ventilation assistée délivrée par un dispositif de ventilation assistée (100) comprenant un circuit d'acheminement de gaz (101), pendant une réanimation cardio-pulmonaire (RCP) pratiquée sur d'un patient (P) en arrêt cardio-pulmonaire, comprenant un boitier autonome (2) destiné à être raccordé au circuit d'acheminement de gaz (101) du dispositif de ventilation assistée (100), et un ensemble de ventilation assistée (1, 100) comprenant un dispositif de ventilation assistée (100) comprenant un circuit d'acheminement de gaz (101), et ledit dispositif de monitorage et de diagnostic (1). Il permet d'établir un diagnostic de l'état ventilatoire du patient de type fermeture des voies aériennes, distension thoracique ou ventilation cible.

## Description

L'invention concerne un dispositif autonome de monitorage d'une ventilation assistée, délivrée par un dispositif de ventilation assistée comprenant un circuit de ventilation, pendant une réanimation cardio-pulmonaire (RCP) pratiquée sur une personne arrêt cardio-pulmonaire, c'est-à-dire un patient, ledit dispositif autonome pouvant être raccordé à des dispositifs de ventilation assistée différents, notamment à un ou des ventilateurs ou appareils d'assistance ventilatoire et/ou à un ou des insufflateurs de gaz manuels.

L'arrêt cardiaque est une cause fréquente de mortalité chez l'homme. La réanimation cardio-pulmonaire (RCP), comprenant le massage cardiaque, pratiquée rapidement sur une personne en arrêt cardiaque permet dans de nombreux cas de sauver cette personne, i.e. patient.

Une RCP comprend une alternance de compressions et relâchements thoraciques pratiqués à une fréquence d'environ 100 à 120 coups/minutes, par un secouriste simultanément à une mise en oeuvre d'une ventilation assistée du patient mise en œuvre par un autre secouriste, par exemple des infirmiers, des pompiers, des médecins-urgentistes ou toute autre personnel soignant.

La ventilation peut être dispensée au moyen d'un insufflateur de gaz manuel, c'est-à-dire un ballon auto-remplisseur à valve unidirectionnelle ou BAVU, ou d'un appareil ou ventilateur d'assistance respiratoire, aussi appelé ventilateur médical, servant à dispenser de l'air ou de l'air enrichi en O₂ au patient.

Toutefois, une ventilation excessive ou insuffisante peut affecter négativement la circulation sanguine du patient, donc nuire au succès de la RCP. Il est donc primordial de pouvoir opérer un monitorage, i.e. un suivi, de la qualité de la ventilation délivrée pendant une RCP, que ce soit avec des systèmes de ventilation simples et/ou manuels de type BAVU, ou plus complexes et/ou automatiques, e.g. les ventilateurs médicaux ou autres appareils d'assistance respiratoire analogues.

Lorsque, pendant une RCP, la ventilation est assurée par un appareil d'assistance respiratoire évolué, i.e. un ventilateur médical, muni d'une turbine ou micro-soufflante fournissant le gaz ou à administration directe de gaz à haute pression, typiquement de l'air et/ou de l'oxygène. Un tel ventilateur médical intègre en général un ou des systèmes spécifiques permettant de mesurer les débits et les pressions des voies aériennes du patient. Ceci est généralement le cas en milieu hospitalier où ce type d'appareil d'assistance respiratoire évolué est disponible.

Cependant, de nombreux patients en arrêt cardiaque, qui sont souvent pris en charge en urgence en dehors des hôpitaux, le sont à l'aide d'un ballon auto-remplisseur à valve unidirectionnelle ou BAVU ou d'un système de ventilation simplifié sans monitorage des paramètres expliqués ci-dessus. En effet, les appareils d'assistance respiratoire évolués ne sont pas transportables et utilisables sur le terrain car trop lourds, encombrants et non conçus pour cela.

Or, lorsqu'un système de ventilation simple et/ou de ventilation manuelle (e.g. BAVU) est utilisé par les secouristes, il ne permet pas, en général, de monitorer les volumes et pressions de gaz car ces systèmes ne comprennent pas de système de monitoring. Il est donc difficile de savoir si un patient ventilé est dans une situation d'hyper ou d'hypoventilation, c'est-à-dire trop ou pas assez de volume gazeux délivré aux poumons du patient. Dans les deux cas, la qualité de la RCP s'en trouve affectée car les deux situations sont délétères. Ainsi, l'hypoventilation engendre des échanges gazeux insuffisants, donc des tissus moins bien oxygénés et du CO₂ difficilement extrait du sang, alors que l'hyperventilation conduit à des pressions intra-thoraciques augmentées, voire excessives, ce qui a pour effet une limitation du retour sanguin veineux vers le cœur.

Le document WO-A-2016/198275 propose un système de monitorage fixé à un insufflateur manuel de type BAVU comprenant un capteur de débit massique pour mesurer les débits gazeux à l'insufflation et à l'exsufflation, une électronique de commande traitant les mesures du capteur, un afficheur et une alimentation électrique. Optionnellement, d'autres capteurs peuvent être prévus. Le boitier se raccorde au capteur qui est lui-même intégré au circuit de gaz reliant le ballon d'insufflation au patient. Dit autrement, le boitier est conçu pour venir se raccorder à un type de capteur de débit spécifique bâtit d'un seul tenant avec un conduit du circuit de gaz, ce qui n'est pas idéal car ce système n'est pas utilisable universellement, c'est-à-dire avec différents appareils ou dispositifs d'insufflation, et sa fabrication est par ailleurs plus compliquée. De plus, le capteur de débit est à usage unique et doit être jeté après chaque utilisation, c'est-à-dire après chaque RCP. Ce n'est pas idéal car engendre une logistique compliquée, des déchets électroniques à recycler et donc des coûts importants. En effet, le boitier devant être couplé au BAVU, sa taille doit être réduite pour ne pas gêner l'utilisation du BAVU en entravant les gestes du secouriste ou en ajoutant un poids important au BAVU manuel. Or, une taille réduite conduit à avoir forcément un afficheur de petite taille, donc limité en termes d'informations affichables, c'est-à-dire n'affichant généralement que des valeurs, ce qui est insuffisant pour suivre correctement une RCP, par exemple une reprise de respiration spontanée du patient avec retour à une circulation sanguine normale ou RACS.

Par ailleurs, US-A-2019/0117930 propose un dispositif de surveillance d'une ventilation assistée d'un patient, notamment au moyen d'un ballon de réanimation. Ce dispositif permet de mesurer la teneur en CO₂ du gaz et de l'afficher sous forme de courbe. Optionnellement, d'autres mesures peuvent être opérées, comme des mesures de pression, de débit ou autre. Toutefois, ce dispositif ne permet pas de proposer un diagnostic ventilatoire, ni d'action à mener.

Le problème est dès lors de proposer un dispositif autonome et universel de monitorage, i.e. de suivi, et de diagnostic permettant de suivre, c'est-à-dire monitorer, un ou plusieurs paramètres ou signaux respiratoires, pendant une RCP pratiquée sur un patient en arrêt cardio-respiratoire, et de proposer un diagnostic et de préférence une action à opérer, lequel soit utilisable sur le terrain en association avec différents appareils de ventilation assistée (i.e. ventilateur médical) ou de ventilation manuelle, (i.e. BAVU ou analogues), simples qui ne comportent pas de moyens de monitorage intégrés. De plus, il doit être possible de réutiliser tout ou la majeure partie du dispositif de monitorage après chaque RCP.

La solution de l'invention porte alors sur un dispositif de monitorage, i.e. de suivi, et de diagnostic adapté pour fonctionner avec un dispositif de ventilation assistée comprenant un circuit d'acheminement de gaz, lors d'une ventilation assistée délivrée par ledit dispositif de ventilation assistée, pendant une réanimation cardio-pulmonaire (RCP) pratiquée sur un patient en arrêt cardio-pulmonaire, ledit dispositif de monitorage et de diagnostic comprenant un boitier autonome destiné à être raccordé au circuit d'acheminement de gaz du dispositif de ventilation assistée, ledit boitier autonome comprenant :
- un passage de gaz interne comprenant une entrée de gaz pour recevoir du gaz provenant du circuit d'acheminement de gaz du dispositif de ventilation assistée, c'est-à-dire lorsqu'un tel dispositif de ventilation assistée est raccordé fluidiquement à l'entrée de gaz,
- des moyens d'aspiration de gaz en communication fluidique avec le passage de gaz pour faire circuler le gaz au sein dudit passage de gaz,
- des moyens de traitement de données comprenant au moins un microprocesseur,
- des moyens de détermination de teneur en CO₂ pour opérer des mesures successives de teneurs en CO₂ du gaz présent dans ledit passage de gaz et fournir aux moyens de traitement de données lesdites mesures successives de teneurs en CO₂,
- des moyens de mesure de pression pour déterminer au moins une pression du gaz et pour fournir au moins une valeur de pression mesurée aux moyens de traitement de données,
- des moyens de détermination de débit ou des moyens de raccordement à des moyens de détermination de débit pour déterminer au moins une valeur de débit de gaz correspondant au débit du gaz dans le circuit d'acheminement de gaz du dispositif de ventilation assistée et/ou fournir auxdits moyens de traitement de données ladite au moins une valeur de débit de gaz, et
- des moyens d'affichage commandés par les moyens de traitement de données.

De plus, selon l'invention, les moyens de traitement de données sont configurés pour :
i) traiter au moins une partie des valeurs de teneurs en CO₂ et éventuellement de pression de gaz et/ou de débit de gaz,
ii) établir au moins un diagnostic de l'état ventilatoire du patient à partir d'au moins une partie des mesures traitées, le diagnostic de l'état ventilatoire étant choisi parmi une fermeture des voies aériennes, une distension thoracique (i.e. volume ventilatoire trop élevé) et une ventilation cible, c'est-à-dire une ventilation adaptée du patient qui est suffisante pour empêcher toute fermeture de ses voies aériennes mais insuffisante pour conduire à toute distension thoracique, et
iii) commander un affichage dudit au moins un diagnostic d'état ventilatoire sur les moyens d'affichage,
   - et les moyens d'affichage sont configurés pour afficher (au moins) ledit au moins un diagnostic d'état ventilatoire.

Dans le cadre de l'invention :
- par « mesure » ou « valeur » d'une grandeur donnée, en particulier de pression, de débit ou de teneur en CO₂, on entend une valeur mesurée ou un signal représentatif d'une valeur mesurée.
- par « état ventilatoire », on entend on entend une indication de qualité de la ventilation assistée opérée sur le patient en arrêt cardiaque au moyen du dispositif de ventilation assistée comprenant le circuit d'acheminement de gaz, c'est-à-dire un ventilateur médical ou un BAVU par exemple.
- par « état circulatoire », on entend une indication de qualité de la réanimation cardiopulmonaire (RCP) avec massage cardiaque (i.e. succession de compressions thoraciques et de relâchements) pratiquée par un ou plusieurs secouristes, i.e. du personnel soignant, sur le patient en arrêt cardiaque.

Selon le mode de réalisation considéré, le dispositif de monitorage et de diagnostic de l'invention peut comprendre l'une ou plusieurs des caractéristiques suivantes :
- les moyens de traitement de données sont en outre configurés pour établir un diagnostic d'état circulatoire du patient à partir d'au moins une partie des mesures traitées.
- les moyens de traitement de données sont en outre configurés pour commander un affichage dudit diagnostic d'état circulatoire sur les moyens d'affichage.
- les moyens d'affichage sont configurés pour afficher ledit diagnostic d'état circulatoire.
- les moyens de traitement de données sont configurés pour établir un diagnostic de l'état ventilatoire et un diagnostic de l'état circulatoire du patient à partir d'au moins une partie des mesures traitées.
- les moyens de mesure de pression permettent de déterminer la pression du gaz régnant dans le passage de gaz interne reflétant la pression au sein du circuit d'acheminement de gaz, donc aussi celle dans les voies aériennes du patient.
- les moyens de traitement de données sont en outre configurés pour commander un affichage sur les moyens d'affichage, d'une courbe en temps réel de CO₂ obtenue à partir de plusieurs mesures successives de teneurs en CO₂ successives traitées, c'est-à-dire un profil de CO₂.
- les moyens d'affichage sont en outre configurés pour afficher ladite courbe en temps réel de CO₂.
- le boitier comprend des moyens de raccordement fluidique destinés à être raccordés fluidiquement à des moyens d'amenée de pression reliés fluidiquement au circuit d'acheminement de gaz du dispositif de ventilation assistée.
- les moyens de mesure de pression sont configurés pour déterminer la pression du gaz alimentant lesdits moyens de raccordement fluidique.
- les moyens d'amenée de pression comprennent une ligne d'amenée de pression, c'est-à-dire un tube, un conduit, de préférence flexible, ou analogue.
- les moyens de traitement de données sont configurés pour traiter des mesures de teneurs en CO₂ et des mesures de pression et/ou de débit de gaz pour établir ledit au moins un diagnostic d'état ventilatoire et/ou d'état circulatoire, c'est-à-dire tout ou partie des mesures opérer pour l'un ou plusieurs de ces paramètres de suivi.
- les moyens de traitement de données sont configurés pour établir un diagnostic de l'état circulatoire du patient comprenant une indication de la manière dont s'effectue le massage cardiaque, par exemple un massage trop fort ou trop faible (i.e. insuffisant), ou un massage trop rapide ou trop lent, ou autre.
- le diagnostic d'état ventilatoire et/ou d'état circulatoire sont obtenus à partir de données mémorisées pouvant se présenter sous différentes formes telles que valeurs cibles, algorithmes obtenus à partir de données cliniques ou autres etc.....
- les moyens de traitement de données sont en outre configurés pour déterminer au moins une recommandation d'action à mener par un personnel soignant en fonction dudit au moins un diagnostic d'état ventilatoire et/ou d'état circulatoire établi, c'est-à-dire une recommandation « ventilatoire » et/ou une recommandation « circulatoire ».
- les moyens de traitement de données sont en outre configurés pour commander un affichage de ladite au moins une recommandation d'action à mener sur les moyens d'affichage, c'est-à-dire la recommandation « ventilatoire » et/ou la recommandation « circulatoire ».
- les moyens d'affichage sont configurés pour afficher la ou les recommandations d'action à mener, c'est-à-dire la recommandation « ventilatoire » et/ou la recommandation « circulatoire ».
- la recommandation d'action à mener, typiquement la recommandation « ventilatoire », est choisie parmi une augmentation de la pression positive, une baisse du volume courant et une continuité de la ventilation (i.e. maintien sans changement).
- la recommandation d'action à mener, typiquement la recommandation « circulatoire », comprend une modification d'un ou plusieurs paramètres qualitatifs reflétant la qualité d'un massage cardiaque choisis parmi une augmentation de la force de compression du massage cardiaque, une diminution de la force de compression du massage cardiaque, une augmentation de la fréquence du massage cardiaque et une diminution de la fréquence du massage cardiaque.
- les moyens de mesure de pression comprennent un capteur de pression.
- les moyens de détermination de débit comprennent un capteur de débit massique ou un capteur de pression différentielle, i.e. à perte de charge.
- les moyens d'aspiration de gaz comprennent une pompe d'aspiration de gaz.
- le circuit d'acheminement de gaz relie l'appareil à une interface respiratoire patient.
- le circuit d'acheminement de gaz comprend un conduit ou passage de gaz.
- le dispositif de monitorage est autonome, c'est-à-dire que le boitier est conçu pour être raccordé à une distance de quelques centimètres à quelques dizaines de centimètres au circuit d'acheminement de gaz du dispositif de ventilation assistée, par exemple entre 10 et 1000 cm, typiquement de l'ordre de 50 cm à 500 cm.
- le passage de gaz interne du boitier comprend un conduit de gaz ou analogue.
- les moyens de détermination de teneur en CO₂ comprennent un capteur de CO₂, i.e. dispositif permettant de déterminer une teneur ou concentration en CO₂.
- les moyens de détermination de teneur en CO₂ comprennent un dispositif de spectrométrie infra-rouge.
- le boitier comprend en outre des moyens de raccordement fluidique destinés à être raccordés fluidiquement à des moyens d'amenée de pression reliés fluidiquement au circuit d'acheminement de gaz du dispositif de ventilation assistée.
- les moyens de mesure de pression sont configurés pour déterminer la pression du gaz alimentant les moyens de raccordement fluidique du boitier.
- les moyens de raccordement fluidique comprennent un connecteur fluidique, par exemple un raccord ou embout de connexion.
- les moyens de traitement de données sont configurés pour traiter ladite au moins une valeur de pression mesurée et commander un affichage, sur les moyens d'affichage, d'au moins une valeur de pression ou une représentation graphique d'au moins une valeur de pression obtenue à partir de ladite au moins une valeur de pression traitée.
- le capteur de pression est configuré pour convertir la pression ou les variations de pression en tension ou variations de tension, ou en intensité ou variation d'intensité électrique, appelée(s) mesure(s) de pression.
- les moyens de détermination de débit sont intégrés au boitier.
- alternativement, le boitier comprend des moyens de raccordement à des moyens de détermination de débit qui sont déportés hors du boitier et qui coopèrent avec le circuit d'acheminement de gaz du dispositif de ventilation assistée.
- le capteur de débit massique forme tout ou partie des moyens de détermination de débit déportés hors du boitier.
- le capteur de débit massique est relié électriquement aux moyens de traitement de données pour leur fournir une ou des mesures de débit, de préférence via un (ou des) câble ou fil électrique ou analogue.
- les moyens de détermination de débit sont configurés pour mesurer le débit de gaz circulant dans les deux sens au sein du circuit de ventilation, c'est-à-dire le gaz allant vers le patient et entrant dans ses poumons pendant les phases de relâchement des contractions thoraciques, et le gaz sortant des poumons du patient pendant les contractions thoraciques.
- alternativement, les moyens de détermination de débit sont configurés pour mesurer le débit de gaz inspiratoire, c'est-à-dire le débit gazeux entrant dans les poumons du patient, par exemple pendant les compressions thoraciques et/ou une ventilation du patient.
- alternativement, les moyens de détermination de débit sont configurés pour mesurer le débit de gaz expiratoire, c'est-à-dire le débit gazeux sortant des poumons du patient, par exemple pendant les compressions thoraciques et/ou une ventilation du patient.
- le boitier comprend un (ou des) connecteur électrique pour permettre une liaison électrique du capteur de débit massique aux moyens de traitement de données intégrés au boitier.
- le capteur de pression différentielle (i.e. à perte de charge) forme tout ou partie des moyens de détermination de débit intégrés au boitier, c'est-à-dire embarqués dans le boitier.
- les moyens de raccordement aux moyens de détermination de débit intégrés au boitier, en particulier à un capteur de pression différentielle intégré au boitier, comprennent une première et une seconde ligne de mesure de pression (i.e. des lignes de prise de pression), et préférentiellement comprennent en outre un premier et un second connecteurs portés par le boitier et configurés pour raccordés fluidiquement les lignes de mesure de pression (i.e. les lignes de prise de pression).
- les moyens d'aspiration de gaz comprennent une pompe d'aspiration de gaz, c'est-à-dire une micro-pompe, par exemple une micro-pompe à membrane.
- le passage de gaz interne est relié fluidiquement à une entrée d'aspiration de la pompe d'aspiration de gaz.
- la pompe d'aspiration de gaz comprend une sortie de refoulement de gaz reliée à l'atmosphère, de préférence via un conduit d'évacuation de gaz et un orifice de sortie porté par le boitier.
- les moyens d'affichage comprennent un écran à affichage numérique, notamment un écran tactile.
- les moyens de traitement de données comprennent (au moins) une carte électronique.
- la (au moins une) carte électronique comprend le microprocesseur, de préférence un microcontrôleur.
- le microprocesseur met en œuvre un ou plusieurs algorithmes.
- la carte électronique comprend les moyens de stockage de données.
- la carte électronique est alimentée en courant électrique.
- il comprend des moyens de fourniture de courant électrique pour fournir du courant électrique à tous les composants nécessitant du courant électrique pour fonctionner.
- les moyens de fourniture de courant électrique comprennent au moins une batterie, notamment rechargeable, intégrée au boitier.
- la batterie est extractible ou non du boitier, de préférence elle est extractible pour pouvoir être rechargée hors boitier, via un chargeur dédié.
- les moyens de fourniture de courant électrique comprennent une liaison secteur (110/220V), par exemple un cordon muni d'une prise de raccordement, permettant d'alimenter le boitier pour fournir du courant électrique aux composants nécessitant du courant électrique pour fonctionner et/ou à la batterie interne pour la recharger lorsqu'elle est complètement ou partiellement vide et/ou a été utilisée par exemple.
- les moyens de fourniture de courant électrique comprennent un transformateur de courant pour réduire la tension et/ou l'intensité du courant, en particulier lorsqu'il provient du secteur (110/220 V).
- les moyens de fourniture de courant électrique alimentent au moins les moyens de traitement de données et les moyens d'affichage, et plus généralement, directement ou indirectement, tous les composants nécessitant de l'électricité pour fonctionner.
- les moyens de traitement de données sont en outre configurés pour commander un affichage sur les moyens d'affichage, d'une valeur de CO₂, de préférence une valeur maximale (Vmax) de CO₂ déterminée sur un intervalle de temps donné, par exemple une durée de 3 à 10 secondes.
- les moyens de traitement de données sont en outre configurés pour commander un affichage sur les moyens d'affichage, d'un volume de gaz calculé à partir du débit de gaz ayant été déterminé ou mesuré.
- les moyens de traitement de données sont configurés pour déterminer un (ou des) volumes de gaz par calcul de l'intégrale du débit de gaz (i.e. du débit en temps réel) sur une période de temps donnée, par exemple une durée de 0.5 à 10 secondes.
- les moyens de traitement de données sont configurés pour commander un affichage, sur les moyens d'affichage, d'au moins une valeur de volume de gaz ou de teneur en CO₂, par exemple une valeur maximale (Vmax) de CO₂, ou une (ou des) représentation graphique d'au moins une desdites valeurs de volume et/ou de teneur en CO₂, par exemple une représentation graphique de type barre-graphe ou autre.
- le boitier est rigide.
- le boitier est en polymère.
- le boitier porte l'écran d'affichage.
- l'écran d'affichage est connecté électriquement aux moyens de traitement de données, en particulier au microprocesseur.
- le boitier comprend en outre un ou plusieurs voyants lumineux, notamment de type LED ou diodes émettrices de lumière.
- le boitier comprend en outre des moyens de stockage de données, en particulier une mémoire de stockage, par exemple de type EEPROM ou autre.
- les moyens de stockage de données connecté électriquement aux moyens de traitement de données, en particulier au microprocesseur.
- le boitier comprend en outre des moyens de sélection, de préférence un ou plusieurs boutons ou touches, actionnables ou activables par un utilisateur.
- le boitier comprend en outre des moyens d'alarme (sonore et/ou visuelle) configurés pour déclencher une alarme, notamment lorsque l'on détecte une pression des voies aériennes trop importante, un débranchement patient, un volume insufflé trop important, des voies aériennes fermées ou autres.
- le boitier comprend en outre des moyens à haut-parleur permettant d'informer l'utilisateur sur l'état ventilatoire du patient (i.e. volume insufflé trop important, voies aériennes fermées...) et/ou en cas de déclenchement d'une alarme.
- le boitier a une longueur comprise entre 10 et 25 cm, une largeur comprise entre 5 et 15 cm, et une hauteur comprise entre 2 et 10 cm.

L'invention porte aussi sur un ensemble de ventilation assistée pour ventiler une personne en arrêt cardio-pulmonaire, pendant une réanimation cardio-pulmonaire (RCP), comprenant :
- un dispositif de ventilation assistée comprenant un circuit d'acheminement de gaz pour acheminer du gaz, puis le fournir aux voies aériennes de la personne en arrêt cardio-pulmonaire, et
- un dispositif de monitorage selon l'invention, comprenant un boitier raccordé au circuit d'acheminement de gaz du dispositif de ventilation assistée au moins par l'intermédiaire :
   o d'un conduit d'amenée de gaz, i.e. une ligne d'acheminement de gaz, reliant fluidiquement le circuit d'acheminement de gaz du dispositif de ventilation assistée à l'entrée de gaz du passage de gaz interne du boitier de manière à alimenter ledit passage de gaz interne en gaz prélevé dans le circuit d'acheminement de gaz du dispositif de ventilation assistée, et
   ∘ d'un conduit d'amenée de pression reliant fluidiquement le circuit d'acheminement de gaz aux moyens de raccordement fluidique du boitier de manière à amener jusqu'auxdits moyens de raccordement fluidique du boitier, la pression gazeuse régnant dans le circuit d'acheminement de gaz du dispositif de ventilation assistée.

Selon le mode de réalisation considéré, l'ensemble de ventilation assistée de l'invention peut comprendre l'une ou plusieurs des caractéristiques suivantes :
- le dispositif de ventilation assistée est choisi parmi les appareils d'assistance ventilatoire, c'est-à-dire les ventilateurs médicaux.
- alternativement, le dispositif de ventilation assistée est choisi parmi les insufflateurs de gaz manuels, c'est-à-dire les BAVU ou analogues.
- le circuit d'acheminement de gaz du dispositif de ventilation assistée comprend un capteur de débit massique agencé pour mesurer le débit de gaz au sein du circuit de ventilation, ledit capteur de débit massique étant connecté électriquement au boitier du dispositif de monitorage pour fournir au moins une valeur de débit mesurée aux moyens de traitement de données via les moyens de raccordement du boitier du dispositif de monitorage.
- alternativement, le circuit d'acheminement de gaz du dispositif de ventilation assistée comprend un premier piquage de pression et un second piquage de pression, séparés l'une de l'autre par un élément à perte de charge agencé dans le circuit de ventilation, et reliés fluidiquement à un capteur de pression différentiel agencé dans le boitier du dispositif de monitorage par l'intermédiaire d'une première et d'une seconde ligne de mesure de pression de manière déterminer un débit de gaz à partir des mesures de pression opérées par le capteur de pression différentiel à partir des pressions provenant des premier et second piquages de pression et acheminées par les première et seconde lignes de mesure de pression.
- le circuit d'acheminement de gaz du dispositif de ventilation assistée comprend au moins un conduit de gaz ou analogue reliant fluidiquement le dispositif de ventilation assistée à une interface respiratoire patient, par exemple un masque respiratoire ou une sonde d'intubation.
- lorsque le dispositif de ventilation assistée est un appareil d'assistance ventilatoire, en particulier un ventilateur médical à micro-soufflante, i.e. turbine ou compresseur, le circuit d'acheminement de gaz est agencé à l'extérieur dudit appareil d'assistance ventilatoire.
- lors que le dispositif de ventilation assistée est un insufflateur manuel de type BAVU ou analogue, comprenant un ballon d'insufflation flexible sur lequel un secouriste vient exercer des pressions manuelles pour en extraire du gaz et l'envoyer vers le patient en arrêt cardio-pulmonaire, le circuit d'acheminement de gaz comprend tout ou partie du ou des éléments de conduit véhiculant le gaz depuis le ballon d'insufflation vers l'interface respiratoire patient, c'est-à-dire situés en aval (i.e. après la sortie) du ballon d'insufflation.
- le circuit d'acheminement de gaz du dispositif de ventilation assistée est relié au boitier du dispositif de monitorage selon l'invention au moyen d'un ou plusieurs d'éléments de raccordement venant se raccorder sur le circuit de ventilation.
- le circuit d'acheminement de gaz du dispositif de ventilation assistée comprend une pièce en Y et une branche inspiratoire et une branche expiratoire reliées fluidiquement aux premières et secondes extrémités de la pièce en Y, la troisième extrémité de la pièce en Y étant raccordée à l'interface respiratoire patient.
- un ou des éléments de raccordement viennent de raccorder fluidiquement à la pièce en Y ou à proximité de la pièce en Y (i.e. < 30 cm).
- le ou les éléments de raccordement comprennent un ou des raccords tubulaires, par exemple un raccords tubulaire équipé d'un filtre.

L'invention va maintenant être mieux comprise grâce à la description détaillée suivante, faite à titre illustratif mais non limitatif, en référence aux figures annexées parmi lesquelles :
- Fig. 1 schématise un premier mode de réalisation d'un ensemble de ventilation assistée selon l'invention comprenant un BAVU et un dispositif autonome de monitorage selon l'invention ;
- Fig. 2 schématise un deuxième mode de réalisation d'un ensemble de ventilation assistée selon l'invention comprenant un BAVU et un dispositif autonome de monitorage selon l'invention ;
- Fig. 3 schématise un troisième mode de réalisation d'un ensemble de ventilation assistée selon l'invention, analogue à celui de la Fig. 2 mais dans lequel le BAVU a été remplacé par un ventilateur médical ; et
- Fig. 4 donne des exemples de courbes de pression, débit et teneur en CO₂ pouvant être affichées par un ensemble de monitorage selon l'invention.

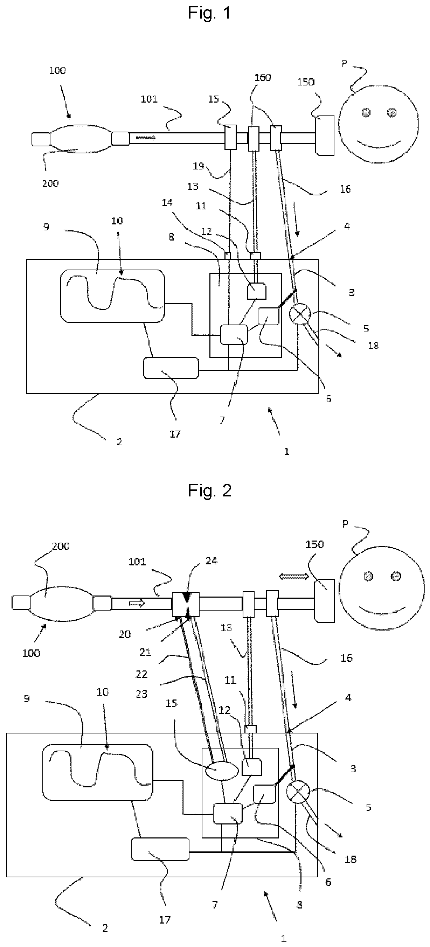

Fig. 1 schématise un premier mode de réalisation d'un ensemble de ventilation assistée 1, 100 selon l'invention, comprenant ici un BAVU 100, et un dispositif autonome de monitorage 1 selon l'invention raccordé au circuit d'acheminement de gaz 101 du BAVU 100, lequel ensemble 1, 100 est destiné à être utilisé pendant une réanimation cardio-pulmonaire ou RCP, pratiquée par des secouristes sur une personne ou patient P en arrêt cardio-pulmonaire.

Le boitier 2 rigide, par exemple en polymère, du dispositif autonome de monitorage 1 selon l'invention est raccordé en plusieurs sites au circuit d'acheminement de gaz 101 du dispositif de ventilation assistée 100, i.e. du BAVU, comme expliqué ci-après.

Le circuit d'acheminement de gaz 101 débouche, en aval, au niveau d'une interface respiratoire patient 150, tel un masque respiratoire, permettant de fournir le gaz provenant du BAVU au patient P.

Le BAVU 100 est normalement actionné par un secouriste pour opérer une ventilation assistée en expulsant le gaz du ballon flexible 200 du BAVU 100, et ainsi fournir le gaz, i.e. de l'air ou de l'air enrichi en O₂, au patient P, alors qu'un autre secouriste pratique un massage cardiaque sur le patient P, c'est-à-dire une RCP comprenant une alternance de compressions et de relâchements thoraciques opérés à une fréquence d'environ 100 à 120 coups/minutes.

Le dispositif de monitorage 1 est autonome, c'est-à-dire qu'il n'est pas intégré au circuit d'acheminement de gaz 101 ou embarqué dans le dispositif de ventilation assistée 100 mais est indépendant et vient se raccorder entre l'interface patient 150 et le BAVU 100.

Il permet de monitorer différents paramètres ou signaux respiratoires, pendant une RCP pratiquée sur le patient P en arrêt cardio-respiratoire, en particulier la teneur en CO₂ du gaz mais aussi préférentiellement le débit gazeux et/ou la pression des voies aériennes, pour en déduire éventuellement un volume gazeux.

Pour ce faire, selon l'invention, le boitier 2 vient se raccorder au circuit d'acheminement de gaz 101 du dispositif de ventilation assistée 100, via un conduit d'amenée de gaz 16, tel un tuyau flexible. Le boitier 2 comprend un passage de gaz 3 interne, tel un conduit ou analogue, avec une entrée de gaz 4 pour recevoir du gaz provenant du conduit d'amenée de gaz 16, donc du circuit d'acheminement de gaz 101 du dispositif de ventilation assistée 100, en particulier du gaz provenant des poumons du patient et expulsé pendant la RCP.

En fait, le gaz est aspiré par des moyens d'aspiration de gaz 5, telle une pompe d'aspiration de gaz, typiquement une micro-pompe à gaz, reliée fluidiquement au passage de gaz 3 de sorte que le gaz puisse remonter jusqu'au boitier 2 depuis le circuit d'acheminement de gaz 101 du dispositif de ventilation assistée 100, via le conduit d'amenée de gaz 16.

Autrement dit, la pompe d'aspiration permet de faire circuler le gaz au sein dudit passage de gaz 3 afin de l'amener à des moyens de détermination de teneur en CO₂ 6 permettant d'opérer des mesures successives de teneurs en CO₂ du gaz au sein dudit passage de gaz 3, c'est-à-dire un capteur de CO₂. La sortie de la pompe d'aspiration 5 est reliée à l'atmosphère via une ligne d'évent 18 pour y rejeter le gaz pompé.

Avantageusement, les moyens de détermination de teneur en CO₂ 6 sont ou comprennent un dispositif de spectrométrie infra-rouge.

Par ailleurs, sont prévus des moyens de traitement de données 7, 8 comprenant au moins un microprocesseur 8 mettant en œuvre un ou des algorithmes, de préférence porté par une carte électronique 7.

Les moyens de détermination de teneur en CO₂ 6 sont configurés pour fournir aux moyens de traitement de données 7, 8, des mesures successives de teneurs en CO₂, et ces derniers 7, 8, en particulier le microprocesseur 8, sont configurés pour traiter les mesures de teneur en CO₂ et commander un affichage sur des moyens d'affichage 9, tel un écran à affichage numérique, par exemple un écran tactile, d'une courbe 10 en temps réel de CO₂ obtenue à partir de plusieurs mesures successives de teneurs en CO₂ successives traitées par le microprocesseur 8.

Autrement dit, les moyens d'affichage 9 sont commandés par les moyens de traitement de données 7, 8 pour afficher la courbe de CO₂ de manière à permettre aux secouristes de suivre l'évolution de la quantité de CO₂ dans les gaz provenant des poumons du patient P, donc de détecter facilement et rapidement un RACS par exemple.

Des moyens de fourniture de courant électrique 17, par exemple une batterie rechargeable, alimente les moyens de traitement de données 7, 8 et les moyens d'affichage 9, ainsi que tous les autres composants nécessitant de l'énergie électrique pour fonctionner.

Préférentiellement, la carte électronique 8 peut aussi comprendre des moyens de stockage de données, telle une mémoire de stockage, par exemple de type EEPROM ou autre. Les moyens de stockage de données enregistrent des données mesurées pendant la RCP, ainsi que des informations ou paramètres s'affichant sur les moyens d'affichage 9 permettant une analyse à posteriori de ces données ou autres.

Par ailleurs, les moyens d'affichage 9 comprennent un afficheur numérique, i.e. un écran, et éventuellement un ou des voyants lumineux, par exemple une ou des LED. Les moyens d'affichage permettent d'afficher la courbe de CO2 mais aussi des valeurs, des messages, des icônes d'alarme..., comme expliqué ci-après.

Par ailleurs, le dispositif autonome de monitorage 1 selon l'invention peut aussi opérer des mesures de débit et/ou de pression afin d'afficher des valeurs de pression, de débit ou de volume à l'attention des secouristes.

En effet, le suivi de ces paramètres ou signaux respiratoires, en plus de la teneur en CO₂, permet d'établir un diagnostic de l'état ventilatoire du patient (P), c'est-à-dire de la ventilation en cours pendant la RCP, à partir de tout ou partie des mesures opérées (i.e. débit, pression, teneur en CO₂) et de proposer une recommandation d'action à mener par le personnel soignant, tel le ou les secouristes pratiquant la RCP sur la patient, en fonction de ce diagnostic d'état ventilatoire établi et ce, afin de permettre une adaptation en temps réel de la ventilation, voire même du massage cardiaque.

Le suivi et le traitement de ces paramètres permettent également un diagnostic de l'état circulatoire du patient, c'est-à-dire du massage cardiaque en cours pendant la RCP, à partir de tout ou partie des mesures opérées (débit, pression, teneur en CO₂...) et de proposer, comme précédemment, une recommandation d'action à mener par le personnel soignant, typiquement le ou les secouristes pratiquant la RCP sur le patient, en fonction de ce diagnostic d'état circulatoire établi et ce, afin de permettre une adaptation en temps réel de la circulation (i.e. massage cardiaque).

Plus précisément, afin de mesurer la pression du gaz dans le circuit d'acheminement de gaz 101 du dispositif de ventilation assistée 100, le boitier 2 comprend en outre des moyens de raccordement fluidique 11, tel qu'un raccord ou embout, pouvant être raccordés fluidiquement à des moyens d'amenée de pression, par exemple une ligne d'amenée de pression 13, tel un conduit flexible, qui viennent se raccorder fluidiquement au circuit d'acheminement de gaz 101 du dispositif de ventilation assistée 100. La ligne d'amenée de pression 13 est reliée fluidiquement au lumen du circuit d'acheminement de gaz 101 de manière à y prélever la pression gazeuse.

Des moyens de mesure de pression 12, tel un capteur de pression, sont aménagés dans le boitier 2 pour déterminer la pression du gaz amenée par la ligne d'amenée de pression 13 qui alimente les moyens de raccordement fluidique 11. Les moyens de mesure de pression 12 fournissent la ou les valeurs de pression mesurées aux moyens de traitement de données 7, 8 où ces valeurs de pression sont traitées. Il est bien entendu que les mesures de pression peuvent être soit des valeurs, soit des signaux, par exemple des tensions, représentatifs ou correspondant à des valeurs.

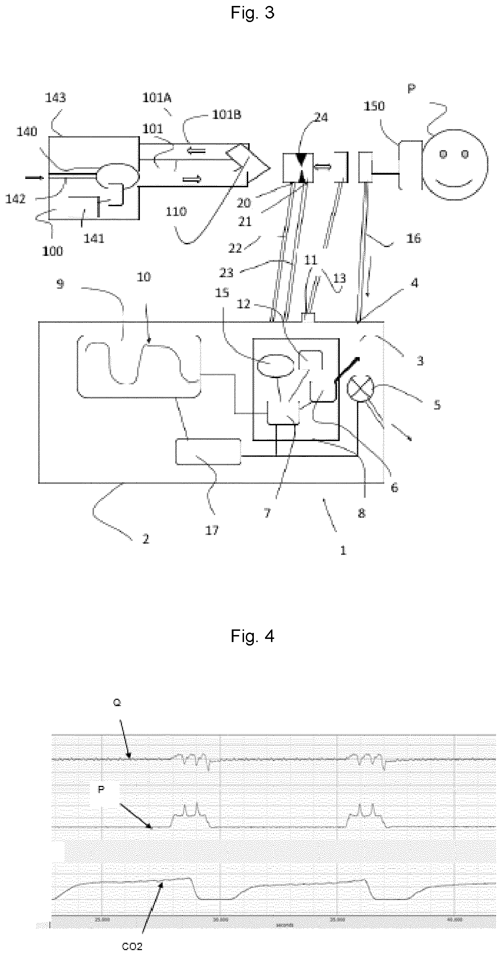

Les moyens de traitement de données 7, 8, qui reçoivent une (des) mesure de pression, sont configurés pour la traiter et ensuite commander éventuellement un affichage, sur les moyens d'affichage 9, de cette valeur de pression ou d'une représentation graphique de cette valeur de pression traitée, par exemple un barre-graphe ou une courbe de pression (P) au fil du temps, comme illustré en Fig. 4.

D'une façon générale, le ligne d'amenée de pression 13 et le conduit d'amenée de gaz 16 viennent se raccorder au circuit d'acheminement de gaz 101 du dispositif de ventilation assistée 100 via un ou des éléments-connecteurs fluidiques 160, par exemples des connecteurs tubulaires venant s'insérer, par exemple par emboitement ou vissage, sur le circuit d'acheminement de gaz 101. L'un des éléments-connecteurs fluidiques 160 peut en outre comprendre un filtre servant à filtrer le gaz et le débarrasser d'éventuels polluants.

De façon analogue, le boitier 2 comprend aussi des moyens de détermination de débit 15 ou des moyens de raccordement 14 à des moyens de détermination de débit 15 pour déterminer et/ou fournir une (ou des) mesure ou valeur de débit de gaz correspondant au débit du gaz dans le circuit d'acheminement de gaz 101 du dispositif de ventilation assistée 100.

Sur Fig. 1, le boitier 2 comprend des moyens de raccordement 14 à des moyens de détermination de débit 15, telle qu'un (des) connecteur électrique pour réaliser une liaison électrique 19 par câbles électriques ou analogue, entre le boitier 2 et les moyens de détermination de débit 15, par exemple un capteur de débit massique déporté, agencés sur le circuit d'acheminement de gaz 101 du dispositif de ventilation assistée 100.

Dans ce cas, les mesures opérées par les moyens de détermination de débit 15, c'est-à-dire le capteur de débit massique, sont amenées par la liaison électrique 19 jusqu'aux moyens de traitement de données 7, 8, qui les reçoivent, puis les traitent, en particulier pour calculer un (des) volumes de gaz par intégration des valeurs de débits sur un intervalle de temps donné, par exemple sur 0,5 à 10 secondes.

Fig. 2 est identique à Fig. 1, à l'exception qu'elle propose un second mode de réalisation dans lequel les moyens de détermination de débit 15 sont directement intégrés, c'est-à-dire embarqués, dans le boitier 2 lui-même. Ces moyens de détermination de débit 15 sont ou comprennent par exemple un capteur de pression différentielle et/ou à perte de charge.

Dans ce cas, le circuit d'acheminement de gaz 101 du dispositif de ventilation assistée 100 comprend un premier 20 et un second 21 piquage de pression, respectivement, séparés l'un de l'autre par un élément à perte de charge 24, telle une restriction de passage, agencé dans le circuit d'acheminement de gaz 101, et reliés fluidiquement au capteur de pression différentiel 15 agencé dans le boitier 2 du dispositif de monitorage 1 par l'intermédiaire d'une première 22 et d'une seconde ligne 23 de mesure de pression, respectivement. Ainsi, on peut déterminer un débit de gaz à partir des mesures de pression opérées par le capteur de pression différentiel 15 à partir des pressions provenant des premier et second piquages de pression 20, 21 et acheminées par les première et seconde lignes de mesure de pression 22, 23.

Cette ou ces mesures de débit de gaz sont transmises aux moyens de traitement de données 7, 8, qui les reçoivent, puis les traitent, en particulier pour calculer un (des) volumes de gaz, comme expliqué ci-avant.

Là encore, les mesures de débit de gaz sont soit des valeurs numériques, soit des signaux représentatifs ou correspondant à de telles valeurs, par exemple une courbe de débit (Q), comme illustré en Fig. 4.

Dans les deux modes de réalisation, les volumes de gaz calculés par moyens de traitement de données 7, 8, en particulier par le microprocesseur, peuvent être affichés sur les moyens d'affichage 9, comme précédemment.

Dans tous les cas, selon l'invention, afin de permettre une adaptation en temps réel de la ventilation, voire même du massage cardiaque, les moyens de traitement de données 7, 8, telle une carte électronique comprenant un (ou des) microprocesseur(s) mettant en œuvre un ou des algorithmes, sont configurés pour :
- traiter tout ou partie des valeurs (i.e. mesures) de pression de gaz, de débit de gaz et de teneurs en CO₂,
- établir un diagnostic de l'état ventilatoire, de l'état circulatoire ou des deux, du patient P à partir des valeurs (i.e. mesures) traitées, et
- ensuite commander un affichage de (ou des) diagnostic d'état ventilatoire et/ou circulatoire sur les moyens d'affichage 9, tel un écran numérique, à destination du personnel soignant.

Ainsi, le diagnostic d'état ventilatoire est préférentiellement une fermeture des voies aériennes, une distension thoracique (donc un volume ventilatoire trop élevé) ou une ventilation cible (i.e. adaptée) du patient qui est suffisante pour empêcher toute fermeture de ses voies aériennes mais insuffisante pour conduire à toute distension thoracique), ou autre.

Par ailleurs, le diagnostic d'état circulatoire comprend préférentiellement une indication de la manière dont s'effectue le massage cardiaque, par exemple massage trop fort ou trop faible (i.e. insuffisant), ou massage trop rapide ou trop lent, ou autre.

Les moyens de traitement de données 7, 8 sont en outre configurés pour déterminer une recommandation d'action à mener par le personnel soignant en fonction du diagnostic d'état ventilatoire et/ou circulatoire qui a été établi, et commander aussi un affichage de la recommandation d'action à mener sur les moyens d'affichage 9.

Par exemple, la recommandation d'action à mener déterminée à partir :
- du diagnostic d'état ventilatoire peut être choisie parmi une augmentation de la pression positive, une baisse du volume courant ou une continuité (i.e. maintien sans changement) de la ventilation, ou autre,
- du diagnostic d'état circulatoire peut être choisie parmi une augmentation de la force de compression du massage cardiaque, une diminution de la force de compression du massage cardiaque, une augmentation de la fréquence du massage cardiaque et une diminution de la fréquence du massage cardiaque, ou autre.

Dès lors, dans le cadre de l'invention, afin d'améliorer ou compléter le suivi de la teneur en CO₂ du gaz, le dispositif de monitorage et de diagnostic de l'invention est configuré pour suivre un et préférentiellement plusieurs autres paramètres ventilatoires, à savoir le débit du gaz, notamment le débit expiratoire du patient, et la pression gazeuse reflétant la pression dans les voies aériennes du patient, voire le volume de gaz qui est l'intégrale du débit sur une durée donnée.

Plus généralement, tous les paramètres ventilatoires suivis permettent aux moyens de traitement de données 7, 8 d'établir un (des) diagnostic d'état ventilatoire et/ou circulatoire et ensuite de proposer une recommandation d'action à mener, lesquels constituent une aide très importante pour les personnels soignants lors des RCP, leur permettant de savoir si la RCP si déroule efficacement ou si des adaptations doivent être opérées.

De plus, en opérant un suivi et un affichage sous forme d'une courbe en temps réel de la teneur en CO₂ des gaz sortant des poumons du patient (i.e. capnométrie) pendant la RCP, on peut encore améliorer ce suivi du déroulé de la RCP car elle donne aux secouristes une indication de bonne ou mauvaise intubation et permet en outre de mesurer la fréquence ventilatoire. Elle inclut aussi une valeur pronostic du patient (i.e. survie ou décès) et facilite la détection du retour à une activité circulatoire spontanée ou RACS et ce, malgré la présence d'oscillations de teneur en CO₂ dans le flux gazeux, provoquées par le massage cardiaque.

Le boitier 2 du dispositif électronique 1 comprend aussi un ou plusieurs boutons ou touches de sélection (non montrés) actionnables par l'utilisateur, par exemple pour allumer ou pour éteindre le dispositif électronique 1 (on/off), pour opérer des sélections ou des choix, pour acquitter des alarmes, pour permettre à l'utilisateur de configurer le dispositif....

Bien entendu, on peut aussi prévoir sur le boitier 2, des moyens de connectivité (non montrés) permettant d'échanger ou de télécharger des données, par exemple un port USB ou autre.

Fig. 3 schématise un troisième mode de réalisation d'un ensemble de ventilation assistée 1 selon l'invention, analogue à celui de la Fig. 2 mais dans lequel le BAVU a été remplacé par un ventilateur médical 100.

Ce ventilateur médical 100 comprend une carcasse externe 143 incorporant, dans le mode de réalisation de Fig. 3, une micro-soufflante motorisée 140 alimentée en air par un conduit d'entrée d'air 142 et commandée par des moyens de pilotage 141, telle une électronique embarquée à microprocesseur. La micro-soufflante motorisée 140 alimente le circuit d'acheminement de gaz 101 en air ou en mélange d'air/oxygène (source d'oxygène non montrée).

Selon un autre mode de réalisation (non montré), le ventilateur 100 ne comporte pas de micro-soufflante mais simplement des alimentations en air et oxygène sous pression, typiquement de l'ordre de quelques bar, par exemple entre 2 et 6 bar.

Ici, le circuit d'acheminement de gaz 101 comprend deux branches respiratoires 101A, 101B agencées en parallèle, à savoir une branche inspiratoire 101A amenant le gaz respiratoire depuis le ventilateur 100 jusqu'au patient P, et une branche expiratoire 101B évacuant les gaz sortant des poumons du patient P. Ces deux branches respiratoires 101A, 101B sont raccordées l'une à l'autre au niveau d'une pièce en Y 110 située entre les deux branches respiratoires 101A, 101B et l'interface respiratoire patient 150. La branche expiratoire 101B est reliée, en aval, au ventilateur 100.

Dans ce cas, les prises 22, 23, 13 des capteurs de pression et débit sont reliées entre la pièce en Y 110 et l'interface patient 150, par exemple un masque respiratoire ou une sonde d'intubation trachéale.

Un exemple d'un affichage de courbes opéré par le microprocesseur des moyens de traitement de données 7, 8 est donné en Fig. 4.

Ces courbes reflètent les variations au fil du temps du débit (Q), de la pression (P) et de la teneur en CO₂ (CO2). Elles permettent au personnel soignant de suivre l'évolution du patient pendant la RCP, notamment de détecter un RACS, un volume insufflé trop important ou une fermeture des voies aériennes du patient.

Ainsi, sur le cycle représenté du signal de CO₂, on ne voit pas de pics dus aux compressions thoraciques (CT), ce qui indique une fermeture des voies aériennes du patient. L'observation physiologique est confirmée par les signaux de pression (P) et de débit (Q) mesurés. Ainsi, on constate que la pression pic à pic des voies aériennes due aux compressions thoraciques est quasi nulle, ce qui signifie que la pression appliquée sur le thorax du patient par les compressions thoraciques n'est pas transmise à la bouche du patient (où se trouve le capteur de pression des voies aériennes). Ceci confirme le phénomène de fermeture des voies aériennes sur le cycle étudié.

L'observation physiologique de fermeture des voies aériennes peut être aussi indiquée ou affichée sur l'écran des moyens d'affichage 6.

D'une façon générale, le dispositif autonome de l'invention permet d'opérer un monitorage efficace d'une ventilation assistée délivrée par un dispositif de ventilation assistée, pendant une réanimation cardio-pulmonaire (RCP) pratiquée sur une personne arrêt cardio-pulmonaire, notamment un ventilateur médical ou appareil d'assistance ventilatoire ou un insufflateur de gaz manuel de type BAVU.

## Revendications

1. Dispositif de monitorage et de diagnostic (1) adapté pour fonctionner avec un dispositif de ventilation assistée comprenant un circuit d'acheminement de gaz (101), lors d'une ventilation assistée délivrée par ledit dispositif de ventilation assistée (100), pendant une réanimation cardio-pulmonaire (RCP), ledit dispositif de monitorage et de diagnostic (1) comprenant un boitier autonome (2) destiné à être raccordé au circuit d'acheminement de gaz (101) du dispositif de ventilation assistée (100), comprenant :
- un passage de gaz (3) interne comprenant une entrée de gaz (4) pour recevoir du gaz provenant du circuit d'acheminement de gaz (101) du dispositif de ventilation assistée (100),
- des moyens d'aspiration de gaz (5) en communication fluidique avec le passage de gaz (3)pour faire circuler le gaz au sein dudit passage de gaz (3),
- des moyens de traitement de données (7, 8) comprenant au moins un microprocesseur (8),
- des moyens de détermination de teneur en CO₂ (6) pour opérer des mesures successives de teneurs en CO₂ du gaz présent dans ledit passage de gaz (3) et fournir aux moyens de traitement de données (7, 8) lesdites mesures successives de teneurs en CO₂,
- des moyens de mesure de pression (12) pour déterminer au moins une pression du gaz et pour fournir ladite au moins une valeur de pression mesurée aux moyens de traitement de données (7, 8),
- des moyens de détermination de débit (15) ou des moyens de raccordement (14) à des moyens de détermination de débit (15) pour déterminer au moins une valeur de débit de gaz correspondant au débit du gaz dans le circuit d'acheminement de gaz (101) du dispositif de ventilation assistée (100) et/ou fournir auxdits moyens de traitement de données (7, 8) ladite au moins une valeur de débit de gaz, et
- des moyens d'affichage (9) commandés par les moyens de traitement de données (7, 8), et dans lequel les moyens de traitement de données (7, 8) sont configurés pour :
i) traiter au moins une partie des valeurs de teneurs en CO₂ et éventuellement de pression de gaz et/ou de débit de gaz,
ii) établir au moins un diagnostic de l'état ventilatoire du patient à partir d'au moins une partie des mesures traitées, le diagnostic de l'état ventilatoire étant choisi parmi une fermeture des voies aériennes, une distension thoracique et une ventilation cible, et
iii) commander un affichage dudit au moins un diagnostic d'état ventilatoire sur les moyens d'affichage (9),
- et les moyens d'affichage (9) sont configurés pour afficher ledit au moins un diagnostic d'état ventilatoire.

2. Dispositif selon la revendication 1, **caractérisé en ce que** :
- les moyens de traitement de données (7, 8) sont en outre configurés pour commander un affichage sur les moyens d'affichage (9), d'une courbe (10) en temps réel de CO₂ obtenue à partir de plusieurs mesures successives de teneurs en CO₂ successives traitées, et
- les moyens d'affichage (9) sont en outre configurés pour afficher ladite courbe (10) en temps réel de CO₂.

3. Dispositif selon la revendication 1, **caractérisé en ce que** le boitier (2) comprend des moyens de raccordement fluidique (11) destinés à être raccordés fluidiquement à des moyens d'amenée de pression reliés fluidiquement au circuit d'acheminement de gaz (101) du dispositif de ventilation assistée (100), les moyens de mesure de pression (12) étant configurés pour déterminer la pression du gaz alimentant lesdits moyens de raccordement fluidique (11).

4. Dispositif selon la revendication 1, **caractérisé en ce que** les moyens de traitement de données (7, 8) sont en outre configurés pour établir un diagnostic d'état circulatoire du patient à partir d'au moins une partie des mesures traitées et pour commander un affichage dudit diagnostic d'état circulatoire sur les moyens d'affichage (9), et les moyens d'affichage (9) étant configurés pour afficher ledit diagnostic d'état circulatoire.

5. Dispositif selon la revendication 1 ou 4, **caractérisé en ce que** les moyens de traitement de données (7, 8) sont configurés pour traiter des mesures de teneurs en CO₂ et des mesures de pression et/ou de débit de gaz pour établir ledit au moins un diagnostic d'état ventilatoire et/ou d'état circulatoire.

6. Dispositif selon la revendication 1 ou 4, **caractérisé en ce que** :
- les moyens de traitement de données (7, 8) sont en outre configurés pour :
• déterminer au moins une recommandation d'action à mener par un personnel soignant en fonction dudit au moins un diagnostic d'état ventilatoire et/ou d'état circulatoire, et
• commander un affichage de ladite au moins une recommandation d'action à mener sur les moyens d'affichage (9), et
- les moyens d'affichage (9) sont configurés pour afficher ladite au moins une recommandation d'action à mener.

7. Dispositif selon la revendication 6, **caractérisé en ce que** ladite au moins une recommandation d'action à mener est choisie parmi :
- une augmentation de la pression positive, une baisse du volume courant ou une continuité de la ventilation, et/ou
- une augmentation ou une diminution de la force de compression du massage cardiaque, et une augmentation ou une diminution de la fréquence du massage cardiaque.

8. Dispositif selon la revendication 1, **caractérisé en ce que** les moyens d'aspiration de gaz (5) comprennent une pompe d'aspiration de gaz.

9. Dispositif selon la revendication 1, **caractérisé en ce que** les moyens de mesure de pression (12) comprennent un capteur de pression.

10. Dispositif selon la revendication 1, **caractérisé en ce que** les moyens de détermination de débit (15) comprennent un capteur de débit massique ou un capteur de pression différentielle.

11. Dispositif selon la revendication 1, **caractérisé en ce que** les moyens de traitement de données (7, 8) sont en outre configurés pour commander un affichage sur les moyens d'affichage (9), d'une valeur de CO₂.

12. Dispositif selon la revendication 11, **caractérisé en ce que** les moyens de traitement de données (7, 8) sont configurés pour commander un affichage sur les moyens d'affichage (9), d'une valeur maximale (Vmax) de CO₂ déterminée sur un intervalle de temps donné compris entre 3 et 10 secondes.

13. Ensemble de ventilation assistée (1, 100) pour ventiler une personne (P) en arrêt cardio-pulmonaire, pendant une réanimation cardio-pulmonaire (RCP), comprenant :
- un dispositif de ventilation assistée (100) comprenant un circuit d'acheminement de gaz (101) pour acheminer du gaz, et
- un dispositif de monitorage et de diagnostic (1) selon l'une des revendications précédentes, comprenant un boitier (2) raccordé au circuit d'acheminement de gaz (101) du dispositif de ventilation assistée (100) au moins par l'intermédiaire :
o d'un conduit d'amenée de gaz (16) reliant fluidiquement le circuit d'acheminement de gaz (101) du dispositif de ventilation assistée (100) à l'entrée de gaz (4) du passage de gaz (3) interne du boitier (2) de manière à alimenter ledit passage de gaz (3) interne en gaz prélevé dans le circuit d'acheminement de gaz (101) du dispositif de ventilation assistée (100), et
o d'un conduit d'amenée de pression (13) reliant fluidiquement le circuit d'acheminement de gaz (101) aux moyens de raccordement fluidique (11) du boitier (2) de manière à amener jusqu'auxdits moyens de raccordement fluidique (11) du boitier (2), la pression gazeuse régnant dans le circuit d'acheminement de gaz (101) du dispositif de ventilation assistée (100).

14. Ensemble de ventilation selon la revendication 13, **caractérisé en ce que** le dispositif de ventilation assistée (100) est choisi parmi les appareils d'assistance ventilatoire.

15. Ensemble de ventilation selon la revendication 13, **caractérisé en ce que** le dispositif de ventilation assistée (100) est choisi parmi les insufflateurs de gaz manuels (BAVU).
